(19) Europäisches Patentamt
European Patent Office
Office européen des brevets

(11) Veröffentlichungsnummer: **0 316 704 B1**

(12) # EUROPÄISCHE PATENTSCHRIFT

(45) Veröffentlichungstag der Patentschrift: **12.07.95**

(51) Int. Cl.[6]: **C07H 19/067**, A61K 31/70

(21) Anmeldenummer: **88118515.1**

(22) Anmeldetag: **07.11.88**

(54) **Fluorcytidinderivate, deren Herstellung und Arzneimittel, die diese Derivate enthalten.**

(30) Priorität: **17.11.87 EP 87116926**

(43) Veröffentlichungstag der Anmeldung:
**24.05.89 Patentblatt 89/21**

(45) Bekanntmachung des Hinweises auf die Patenterteilung:
**12.07.95 Patentblatt 95/28**

(84) Benannte Vertragsstaaten:
**AT BE CH DE ES FR GB GR IT LI LU NL SE**

(56) Entgegenhaltungen:
**CH-A- 633 810**
**FR-A- 2 140 526**

**THE JOURNAL OF MEDICINAL CHEMISTRY, Band 22, Nr. 11, November 1979, Seiten1330-1335, American Chemical Society; A.F. COOK et al.: "Fluorinated pyrimidinenucleosides. 3. Synthesis and antitumor activity of a series of 5'-deoxy-5-fluoropyrimidine nucleosides**

(73) Patentinhaber: **F. HOFFMANN-LA ROCHE AG**
**Postfach 3255**
**CH-4002 Basel (CH)**

(72) Erfinder: **Fujiu, Morio**
**Murota 2-4-3, Chigasaki-shi**
**Kanagawa-ken (JP)**
Erfinder: **Ishitsuka, Hideo**
**Katsura-cho 1-1-1-405**
**Sakae-ku**
**Yokohama-shi**
**Kanagawa-ken (JP)**
Erfinder: **Miwa, Masanori**
**Kamakura Green Mansion 404**
**Ueki 436-1**
**Kamakura-shi**
**Kanagawa-ken (JP)**
Erfinder: **Umeda, Isao Imperial Higashihakura-ku**
**Garden House B-513**
**Shirahata minami1-1**
**Kanagawa-ku**
**Yokohama-shi**
**Kanagawa-ken (JP)**
Erfinder: **Yokose, Kazuteru**
**Nekozane 3-19-16, Urayasu-shi**
**Chiba-ken (JP)**

(74) Vertreter: **Lederer, Franz, Dr. et al**
**Lederer, Keller & Riederer**
**Patentanwälte**
**Prinzregentenstrasse 16**
**D-80538 München (DE)**

EP 0 316 704 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 5'-Deoxy-5-fluorcytidinderivate, ein Verfahren zur Herstellung davon und Antitumormittel auf der Basis dieser Derivate.

Letztere haben die allgemeine Formel

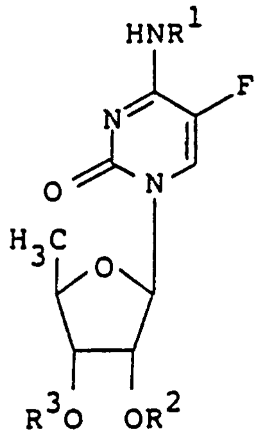

(I)

worin $R^1$, $R^2$ und $R^3$ unabhängig voneinander Wasserstoff oder eine unter physiologischen Bedingungen leicht abspaltbare Gruppe sind, mit der Bedingung, dass zumindest eines von $R^1$, $R^2$ und $R^3$ eine unter physiologischen Bedingungen leicht abspaltbare Gruppe ist, sowie Hydrate oder Solvate der Verbindungen der Formel I, wobei die leicht hydrolysierbaren Gruppen $R^1$, $R^2$ und $R^3$ in der Formel I eine der Formel

$R^4CO$-, $R^5OCO$- oder $R^6SCO$-

worin $R^4$ Wasserstoff, Alkyl, Cycloalkyl, Oxoalkyl, Alkenyl, Aralkyl oder Aryl und $R^5$ und $R^6$ Alkyl oder Aralkyl sind.

Im Rahmen der vorliegenden Erfindung bezeichnet "Alkyl" eine geradkettige oder verzweigte Kette mit 1-19 C-Atomen, z.B. Methyl, Aethyl, Propyl, Isopropyl, Butyl, Isobutyl, sek.-Butyl, t-Butyl, Pentyl, Isopentyl, Neopentyl, Hexyl, Isohexyl, Heptyl, Octyl, Nonyl, Decyl, Undecyl, Dodecyl, Tridecyl, Tetradecyl, Pentadecyl, Hexadecyl, Heptadecyl oder Nonadecyl. "Cycloalkyl" bedeutet z.B. Cyclopropyl, Cyclobutyl, Cyclopentyl, Cyclohexyl oder Adamantyl. "Oxoalkyl" bezeichnet z.B. Acetyl, Propionyl, Butyryl, 2-Oxopropyl oder 3-Oxobutyl. "Alkenyl" bezeichnet gegebenenfalls substituiertes Alkenyl mit 3-19 C-Atome, wie Allyl, Butenyl, 3-Methyl-2-butenyl, 1-Methyl-2-propenyl, Hexenyl, Decenyl, Undecenyl, Tridecenyl, Pentadecenyl, Heptadecenyl, Heptadecadienyl, Heptadecatrienyl, Nonadecenyl, Nonadecadienyl, Nonadecatetraenyl oder 2-Phenylvinyl. "Aralkyl" bedeutet gegebenenfalls substituiertes Aralkyl, wie Benzyl, 1-Phenyläthyl, Methyl-, Fluor-, Chlor-, Methoxy-, Dimethoxy- oder Nitrobenzyl, Phenäthyl, Picolyl oder 3-Indolylmethyl. "Aryl" bezeichnet gegebenenfalls substituiertes Aryl, wie Phenyl, Tolyl, Xylyl, Mesityl, Cumenyl, Aethyl-, Fluor-, Chlor-, Brom-, Jod-, Difluor-, Dichlor-, Methoxy-, Dimethoxy-, Trimethoxy-, Aethoxy-, Diäthoxy-, Triäthoxy-, Propoxy-, Methylendioxy-, Methylthio-, Nitro-, Cyan-, Acetyl- oder Carbamoylphenyl, Methoxycarbonylphenyl, Naphthyl, Biphenylyl, Thienyl, Methylthienyl, Furyl, Nitrofuryl, Pyrrolyl, Methylpyrrolyl, Imidazolyl, Pyrazolyl, Pyridyl, Methylpyridyl oder Pyrazinyl.

Besonders bevorzugte Verbindungen der Formel I sind die folgenden:

$N^4$-Acetyl-5'-deoxy-5-fluorcytidin,
5'-Deoxy-5-fluor-$N^4$-propionylcytidin,
$N^4$-Butyryl-5'-deoxy-5-fluorcytidin,
5'-Deoxy-5-fluor-$N^4$-isobutyrylcytidin,
5'-Deoxy-5-fluor-$N^4$-(2-methylbutyryl)cytidin,
5'-Deoxy-$N^4$-(2-äthylbutyryl)-5-fluorcytidin,
5'-Deoxy-$N^4$-(3,3-dimethylbutyryl)-5-fluorcytidin,
5'-Deoxy-5-fluor-$N^4$-pivaloylcytidin,
5'-Deoxy-5-fluor-$N^4$-valerylcytidin,
5'-Deoxy-5-fluor-$N^4$-isovalerylcytidin,
5'-Deoxy-5-fluor-$N^4$-(2-methylvaleryl)cytidin,
5'-Deoxy-5-fluor-$N^4$-(3-methylvaleryl)cytidin,

5'-Deoxy-5-fluor-$N^4$-(4-methylvaleryl)cytidin,
5'-Deoxy-5-fluor-$N^4$-hexanoylcytidin,
5'-Deoxy-5-fluor-$N^4$-heptanoylcytidin,
5'-Deoxy-5-fluor-$N^4$-octanoylcytidin,
5'-Deoxy-5-fluor-$N^4$-nonanoylcytidin,
5'-Deoxy-5-fluor-$N^4$-hexadecanoylcytidin,
$N^4$-Benzoyl-5'-deoxy-5-fluorcytidin,
5'-Deoxy-5-fluor-$N^4$-(4-methylbenzoyl)cytidin,
5'-Deoxy-5-fluor-$N^4$-(3-methylbenzoyl)cytidin,
5'-Deoxy-5-fluor-$N^4$-(2-methylbenzoyl)cytidin,
5'-Deoxy-$N^4$-(4-äthylbenzoyl)-5-fluorcytidin,
5'-Deoxy-$N^4$-(3,4-dimethylbenzoyl)-5-fluorcytidin,
5'-Deoxy-$N^4$-(3,5-dimethylbenzoyl)-5-fluorcytidin,
5'-Deoxy-5-fluor-$N^4$-(4-methoxybenzoyl)cytidin,
5'-Deoxy-$N^4$-(3,4-dimethoxybenzoyl)-5-fluorcytidin,
5'-Deoxy-$N^4$-(3,5-dimethoxybenzoyl)-5-fluorcytidin,
5'-Deoxy-5-fluor-$N^4$-(3,4,5-trimethoxybenzoyl)cytidin,
5'-Deoxy-5-fluor-$N^4$-(3,4,5-triäthoxybenzoyl)cytidin,
5'-Deoxy-$N^4$-(4-äthoxybenzoyl)-5-fluorcytidin,
5'-Deoxy-5-fluor-$N^4$-(4-propoxybenzoyl)cytidin,
5'-Deoxy-$N^4$-(3,5-diäthoxybenzoyl)-5-fluorcytidin,
$N^4$-(4-Chlorbenzoyl)-5'-deoxy-5-fluorcytidin,
5'-Deoxy-$N^4$-(3,4-dichlorbenzoyl)-5-fluorcytidin,
5'-Deoxy-$N^4$-(3,5-dichlorbenzoyl)-5-fluorcytidin,
5'-Deoxy-5-fluor-$N^4$-(4-nitrobenzoyl)cytidin,
5'-Deoxy-5-fluor-$N^4$-(4-methoxycarbonylbenzoyl)cytidin,
$N^4$-(4-Acetylbenzoyl)-5'-deoxy-5-fluorcytidin,
5'-Deoxy-5-fluor-$N^4$-(phenylacetyl)cytidin,
5'-Deoxy-5-fluor-$N^4$-(4-methoxyphenylacetyl)cytidin,
5'-Deoxy-5-fluor-$N^4$-nicotinoylcytidin,
5'-Deoxy-5-fluor-$N^4$-isonicotinoylcytidin,
5'-Deoxy-5-fluor-$N^4$-picolinoylcytidin,
5'-Deoxy-5-fluor-$N^4$-(2-furoyl)cytidin,
5'-Deoxy-5-fluor-$N^4$-(5-nitro-2-furoyl)cytidin,
5'-Deoxy-5-fluor-$N^4$-(2-thenoyl)cytidin,
5'-Deoxy-5-fluor-$N^4$-(5-methyl-2-thenoyl)cytidin,
5'-Deoxy-5-fluor-$N^4$-(1-methyl-2-pyrrolcarbonyl)cytidin,
5'-Deoxy-5-fluor-$N^4$-(3-indolylacetyl)cytidin,
$N^4$-(3-Butenoyl)-5'-deoxy-5-fluorcytidin,
3'-O-Benzoyl-5'-deoxy-5-fluorcytidin,
$N^4$,3'-O-Dibenzoyl-5'-deoxy-5-fluorcytidin und
5'-Deoxy-$N^4$-(äthylthio)carbonyl-5-fluorcytidin.

Weitere bevorzugte Verbindungen der Formel I sind die folgenden:

5'-Deoxy-5-fluor-$N^4$-octadecanoylcytidin,
$N^4$-Cyclopropancarbonyl-5'-deoxy-5-fluorcytidin,
$N^4$-Cyclohexancarbonyl-5'-deoxy-5-fluorcytidin,
$N^4$-(1-Adamantancarbonyl)-5'-deoxy-5-fluorcytidin,
5'-Deoxy-5-fluor-$N^4$-(2-methoxybenzoyl)cytidin,
5'-Deoxy-$N^4$-(2,4-dimethoxybenzoyl)-5-fluorcytidin,
5'-Deoxy-5-fluor-$N^4$-piperonyloylcytidin,
5'-Deoxy-5-fluor-$N^4$-(4-fluorbenzoyl)cytidin,
$N^4$-(2-Chlorbenzoyl)-5'-deoxy-5-fluorcytidin,
$N^4$-(3-Chlorbenzoyl)-5'-deoxy-5-fluorcytidin,
5'-Deoxy-5-fluor-$N^4$-(3-nitrobenzoyl)cytidin,
5'-Deoxy-5-fluor-$N^4$-[4-(methylthio)benzoyl]cytidin,
5'-Deoxy-5-fluor-$N^4$-(2-naphthoyl)cytidin,
5'-Deoxy-5-fluor-$N^4$-(3-furoyl)cytidin,
5'-Deoxy-5-fluor-$N^4$-(3-phenylpropionyl)cytidin,

$N^4$-Cinnamoyl-5'-deoxy-5-fluorcytidin,
2',3'-di-O-Benzoyl-5'-deoxy-5-fluorcytidin,
$N^4$,2'-0,3'-O-Tribenzoyl-5'-deoxy-5-fluorcytidin,
5'-Deoxy-5-fluor-$N^4$-(octyloxycarbonyl)cytidin,
$N^4$-(Benzyloxycarbonyl)-5'-deoxy-5-fluorcytidin und
5'-Deoxy-5-fluor-$N^4$-formylcytidin.

Die Verbindungen der Formel I und die Hydrate und Solvate davon können dadurch hergestellt werden, dass man eine Verbindung der allgemeinen Formel

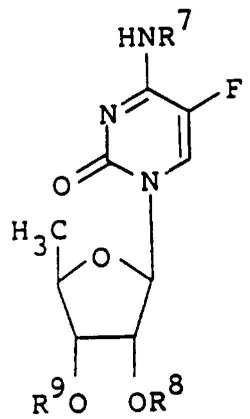

(II)

worin $R^7$ Wasserstoff oder eine Aminoschutzgruppe, $R^8$ und $R^9$ unabhängig voneinander Wasserstoff oder eine Hydroxyschutzgruppe oder $R^8$ und $R^9$ zusammen eine cyclische Hydroxyschutzgruppe sind, mit einer Verbindung der allgemeinen Formel $XCOR^4$, worin X eine Abgangsgruppe und $R^4$ Wasserstoff, Alkyl, Cycloalkyl, Oxoalkyl, Alkenyl, Aralkyl oder Aryl ist, oder mit einer Verbindung der allgemeinen Formel $YCOR^{10}$, worin Y Halogen und $R^{10}$ eine Gruppe der Formel $R^5O$- oder $R^6S$-, in der $R^5$ und $R^6$ Alkyl oder Aralkyl sind, umsetzt und eine vorhandene Schutzgruppe abspaltet.

Beispiele von Aminoschutzgruppen sind Benzyloxycarbonyl, Phenoxycarbonyl, 2,2-Trichloräthoxycarbonyl, Aethoxycarbonyl, t-Butoxycarbonyl und Trifluoracetyl. Beispiele von Hydroxyschutzgruppen sind Benzyl, Methoxybenzyl, Trimethylsilyl, Triäthylsilyl, Isopropyldimethylsilyl, t-Butyldimethylsilyl, t-Butyldiphenylsilyl, Thexyldimethylsilyl, Allyl, Methoxymethyl, (2-Methoxyäthoxy)methyl und Tetrahydropyranyl. Beispiele von cyclischen Hydroxyschutzgruppen sind cyclische Acetale, Ketale, Carbonate oder Orthoester oder cyclische 1,3-(1,1,3,3-Tetraisopropyl)disiloxandiyl-Derivate. Beispiele von Abgangsgruppen sind Halogene, Acyloxy, Alkyloxycarbonyloxy, Succinimidoxy, Phthalimidoxy, 4-Nitrophenyl, Azido, 2,4,6-Triisopropylbenzolsulfonyl und Diäthoxyphosphoryloxy. "Halogen" bezeichnet Chlor, Brom oder Jod.

Unter den Verbindungen der Formel II ist 5'-Deoxy-5-fluorcytidin bekannt [J. Med. Chem., 22, (1979) 1330]. Die anderen Verbindungen der Formel II können in an sich bekannter Weise [Chem. Pharm. Bull., 33, (1985) 2575] ausgehend von 5'-Deoxy-5-fluorcytidin oder von 5'-Deoxy-5-fluoruridin hergestellt werden.

Die im obigen Verfahren verwendete Verbindungen der Formel $XCOR^4$ sind Säurehalogenide, Säureanhydride, gemischte Anhydride (hergestellt durch Reaktion von $R^4COOH$ mit 2,4,6-Triisopropylbenzolsulfonylchlorid oder Diäthylchlorphosphat, worin $R^4$ die obige Bedeutung hat) aktivierte Ester, wie N-Hydroxysuccinimidester, N-Hydroxyphthalimidester oder 4-Nitrophenylester; Acylazide oder gemischte Carbonsäureanhydride. Die Verbindungen der Formel $YCOR^{10}$ sind Alkoxycarbonylhalogenide, Aralkoxycarbonylhalogenide, Alkylthiocarbonylhalogenide oder Aralkylthiocarbonylhalogenide.

Die Reaktion einer Verbindung der Formel II mit einer Verbindung der Formel $XCOR^4$ oder $YCOR^{10}$ kann man in einem Lösungsmittel, wie Pyridin, Dioxan, Tetrahydrofuran, Acetonitril, Chloroform, Dichlormethan, Methanol, Aethanol oder Wasser oder Gemische davon durchführen, in Gegenwart eines Säureakzeptors, wie Triäthylamin, Pyridin, Picolin, Dimethylaminopyridin, Lutidin, N,N-Dimethylanilin oder eines Alkalimetallhydroxids, -carbonats oder -phosphats. Die Reaktion kann in einem breiten Temperaturbereich durchgeführt werden, zweckmässig jedoch zwischen etwa 0 und 120 °C, vorzugsweise zwischen 0 und 50 °C. Für 1 Mol einer Verbindung der Formel II werden 1, 2 oder 3 Mol oder ein Ueberschuss einer Verbindung der Formel $XCOR^4$ oder $YCOR^{10}$ verwendet.

Die Abspaltung einer Schutzgruppe kann in an sich bekannter Weise durchgeführt werden.

Die Verbindungen der vorliegenden Erfindung können in an sich bekannter Weise isoliert und gereinigt werden, z.B. durch Abdampfen, Filtrierung, Extraktion, Abfällen, Chromatographie und/oder Umkristallisation.

Die Verbindungen der Formel I können als Solvate, insbesondere als Hydrate vorliegen. Die Hydrierung kann im Laufe der Herstellung oder als Resultat der hygroskopischen Eigenschaften eines ursprünglich wasserfreien Produktes erfolgen. Zur Herstellung eines Hydrats kann ein vollständig oder teilweise wasserfreies Produkt, z.B. bei etwa 10 bis 40 °C einer feuchten Atmosphäre ausgesetzt werden. Solvate mit pharmazeutisch anwendbaren Lösungsmitteln, wie Aethanol, können z.B. während einer Kristallisation entstehen.

Die Verbindungen der Formel I sowie die Hydrate und Solvate davon sind gegen Sarcoma 180, Meth A Fibrosarcoma und Lewis Lungenkarzinom bei Mäusen über einen breiten Dosierungsbereich sowohl oral wie parenteral wirksam und können als Antitumormittel Verwendung finden. 5-Fluoruracil und seine Derivate haben den Nachteil der intestinalen und immunosuppressiven Toxizität, was ihre Verwendung drastisch limitiert. Bei oraler Verabreichung sind die vorliegenden Verbindungen der Formel I viel weniger toxisch im intestinalen Trakt und für das Immunsystem als 5-Fluoruracil (J.A.C.S. 79, 1957, 4559) und die Vorläufer davon, wie Tegafur: Uracil, 1:4 (UFT) und 5′-Deoxy-5-fluoruridin (US 4071680). Daher können die Verbindungen der Formel I bei der Behandlung von verschiedenen Tumoren beim Menschen Verwendung finden.

Die Erfindung betrifft ebenfalls pharmazeutische Präparate, die eine oder mehrere Verbindungen der Formel I enthalten, sowie die Verwendung dieser Verbindungen zur Herstellung von Arzneimitteln zur Behandlung von Tumoren.

Die Verbindungen der Formel I kann man dem Menschen oral oder nicht oral verabreichen, gegebenenfalls unter Zusatz eines verträglichen Trägermaterials. Letzteres kann eine für die enterale, perkutane oder parenterale Verabreichung geeignete organische oder anorganische inerte Substanz sein, wie Wasser, Gelatine, Gummi arabicum, Lactose, Stärke, Magnesiumstearat, Talk, pflanzliche Oele oder Polyalkylenglykole. Die pharmazeutischen Präparate können in fester Form als Tabletten, Dragées, Granulate, Kapseln, Suppositorien oder als enteral verabreichbare Tabletten, Granulate oder Kapseln; in halbfester Form als Salben oder in flüssiger Form als Lösungen, Suspensionen oder Emulsionen vorliegen. Sie können sterilisiert sein und/oder weitere Hilfsmittel, wie Konservierungs- oder Stabilisierungsmittel, Emulgatoren, Riechstoffe, Salze zur Veränderung des osmotischen Druckes oder Puffer enthalten. Sie können in an sich bekannter Weise hergestellt werden.

Die Verbindungen der Formel I können einzeln oder als Gemische von zwei oder mehr Verwendung finden, wobei der Gehalt an Wirkstoff im Bereich von etwa 0,1 bis 99,5%, vorzugsweise 0,5 bis 95% des Gesamtgewichts des Präparats, liegt. Die Präparate können auch weitere pharmazeutisch wirksame Substanzen enthalten.

Die tägliche Dosis der Verbindungen der Formel I hängt von dem Gewicht des Patienten und der zu behandelnden Krankheit ab, liegt jedoch im allgemeinen im Bereich von 0,5 bis 700, vorzugsweise von etwa 3 bis 500 mg/kg Körpergewicht. Die Antitumorwirksamkeit der Verbindungen der Formel I wird in den folgenden Versuchen veranschaulicht:

Antitumorwirkung gegen Sarcoma 180

Sarcoma 180 Zellen ($2 \times 10^6$ Zellen) werden am Tag 0 Mäusen (20-22 g) subkutan implantiert. Die Testsubstanzen werden täglich vom Tag 1 bis zum Tag 7 oral verabreicht. Die Tiere werden am Tag 14 getötet und die Tumoren werden herausgenommen und gewogen. Die in der nachfolgenden angegebene Hemmung des Tumorwachstums in Tabelle 1 wird wie folgt ermittelt:

$$\% \text{ Hemmung} = (1 - T/C) \times 100$$

T = Gewicht der Tumoren der behandelten Gruppe
C = Gewicht der Tumoren der Kontrollgruppe.

Tabelle 1

| Antitumorwirksamkeit gegen Sarcoma 180 bei Mäusen | | |
|---|---|---|
| Verbindung (Beispiel-Nr.) | Dosis x 7 (mMol/kg/Tag) | Hemmung (%) |
| 1 | 1,1<br>2,2 | 56<br>83 |
| 2 | 1,5<br>3,0 | 84<br>91 |
| 5 | 1,1<br>2,2 | 62<br>82 |
| 6 | 0,5<br>1,5 | 28<br>76 |
| 7 | 1,4<br>2,7 | 57<br>84 |
| 8 | 1,4<br>2,7 | 20<br>77 |
| 9 | 1,4<br>2,7 | 76<br>96 |
| 10 | 1,5<br>3,0 | 74<br>97 |
| 11 | 0,8<br>1,5 | 69<br>90 |
| 12 | 0,8<br>1,5 | 40<br>73 |
| 13 | 0,8<br>1,5 | 28<br>66 |
| 15 | 0,8<br>1,5 | 47<br>62 |
| 17 | 1,3<br>2,6 | 75<br>92 |
| 24 | 1,5<br>3,0 | 63<br>94 |
| 41 | 0,5<br>1,5 | -18<br>36 |
| 42 | 0,5<br>1,5 | 0<br>36 |

Antitumorwirksamkeit gegen Meth A Fibrosarcoma

Meth A Fibrosarcoma Zellen (2 x $10^5$ Zellen) werden Mäusen (21-22 g) subkutan implantiert. Der Versuch gegen Meth A Fibrosarcoma und die Ermittlung der Hemmung des Tumorwachstums wurde in ähnlicher Weise wie beim Versuch gegen Sarcoma 180 durchgeführt. Die Resultate sind in der Tabelle 2 angegeben.

In ähnlicher Weise wurde die Antitumorwirksamkeit der Verbindung des vorliegenden Beispiels 3 mit derjenigen von 5′-Deoxy-5-fluoruridin verglichen. Die Resultate sind in der Tabelle 3 angegeben. Am Tag 8 des Versuchs wurde der Kot beobachtet. Die Resultate zeigen, dass die Verbindung des Beispiels 3 höhere eine Antitumorwirksamkeit und eine niedrigere Toxizität als 5′-Deoxy-5-fluoruridin aufweist. Im gleichen Experiment verursachte die Verbindung des Beispiels 3 keine Diarrhea während letztere der die Dosis

limitierende Faktor von 5'-Deoxy-5-fluoruridin darstellt.

Tabelle 2

Antitumorwirksamkeit gegen Meth A Fibrosarcoma bei Mäusen

| Verbindung (Beispiel-Nr.) | Dosis x 7 (mMol/kg/Tag) | Hemmung (%) |
|---|---|---|
| 1 | 1,5 | 50 |
| | 3,0 | 72 |
| 3 | 1,5 | 86 |
| | 3,0 | 79 |
| 14 | 1,5 | 66 |
| | 3,0 | 94 |
| 16 | 0,8 | 38 |
| | 1,5 | 58 |
| 18 | 1,5 | 51 |
| | 3,0 | 91 |
| 19 | 1,5 | 3 |
| | 3,0 | 64 |
| 20 | 1,5 | 53 |
| | 3,0 | 84 |
| 21 | 0,8 | 17 |
| | 1,5 | 60 |
| 22 | 1,5 | 42 |
| | 3,0 | 42 |
| 23 | 0,8 | 56 |
| | 1,5 | 64 |
| 25 | 1,5 | - 6 |
| | 3,0 | 34 |
| 26 | 1,5 | 37 |
| | 3,0 | 58 |
| 27 | 1,5 | 58 |
| | 3,0 | 91 |
| 28 | 1,5 | -13 |
| | 3,0 | -13 |
| 29 | 1,5 | 49 |
| | 3,0 | 92 |
| 30 | 1,5 | 55 |
| | 3,0 | 58 |
| 31 | 1,5 | 55 |
| | 3,0 | 84 |
| 34 | 1,3 | 75 |
| | 2,6 | 92 |
| 36 | 1,5 | 53 |
| | 3,0 | 92 |
| 37 | 1,5 | 59 |
| | 3,0 | 86 |

Tabelle 2 (Fortsetzung)

| Verbindung (Beispiel-Nr.) | Dosis x 7 (mMol/kg/Tag) | Hemmung (%) |
|---|---|---|
| 40 | 0,8 | 41 |
| | 1,5 | 57 |
| 44 | 1,5 | 38 |
| | 3,0 | 66 |
| 45 | 1,5 | 49 |
| | 3,0 | 71 |
| 46 | 1,5 | 51 |
| | 3,0 | 66 |
| 47 | 1,5 | 29 |
| | 3,0 | 59 |
| 48 | 0,8 | 42 |
| | 1,5 | 72 |
| 49 | 1,5 | 58 |
| | 3,0 | 76 |
| 52 | 0,8 | 41 |
| | 1,5 | 51 |
| 53 | 1,5 | 48 |
| | 3,0 | 85 |
| 54 | 1,5 | 55 |
| | 3,0 | 85 |
| 57 | 1,5 | 28 |
| | 3,0 | 56 |
| 59 | 1,5 | 23 |
| | 3,0 | 80 |

Tabelle 3

| Antitumorwirksamkeit gegen Meth A Fibrosarcoma bei Mäusen und Beobachtung des Kots am Tag 8 | | | |
|---|---|---|---|
| Verbindung (Beisp.-Nr.) | Dosis x 7 (mMol/kg/Tag) | Hemmung (%) | Fekale Beobachtung* |
| 3 | 0.4 | -27 | N |
| | 0.8 | 20 | N |
| | 1.5 | 86 | N |
| | 3.0 | 79 | N |
| 5'-Deoxy-5-fluoruridin | 0.4 | 34 | N |
| | 0.8 | 31 | N |
| | 1.5 | 66 | L - D |
| | 3.0 | toxisch | D |

* Fekale Beobachtung
N: normaler Kot
L: loser Kot
D: Diarrhea

Antitumorwirksamkeit gegen Lewis-Lungenkarzinom

Die Antitumorwirksamkeit der Verbindung des vorliegenden Beispiels 1 wurde mit derjenigen von 5'-Deoxy-5-fluoruridin und des Kombinationspräparats, UFT (Tegafur: Uracil, 1:4) verglichen. Mäusen wurde am Tag 0 Lewis-Lungenkarzinom ($10^6$ Zellen) subkutan inokuliert. Die Verbindungen wurden täglich 14 mal ab Tag 1 oral verabreicht. Die effektive Dosis ($ED_{50}$), bei der das Wachstum des Tumors um 50% gehemmt wurde und die toxischen Dosen wurden ermittelt. Der therapeutische Index (toxische Dose/$ED_{50}$) sind in der Tabelle 4 angegeben. Die Resultate zeigen, dass die Verbindung des Beispiels 1 einen höheren therapeutischen Index hat als die klassischen Vorläufer von 5-Fluoruracil, 5'-Deoxy-5-fluoruridin und UFT. Sie ist weniger toxisch im intestinalen Trakt (Diarrhea) und in den für die Immunität verantwortlichen Organe (Thymus und Knochenmark) und weist daher einen höheren Sicherheitsgrad auf.

Tabelle 4

| Verbindung (Beispiel Nr.) | Therapeutischer Index (Toxische Dose/$ED_{50}$ *) | | | |
|---|---|---|---|---|
| | Max. Dosis ohne Diarrhea (A)** | 10% Körpergewicht Abnahme (B)** | 50% Thymus-Einschrumpfung (C)** | 50% Reduktion der Anzahl Zellen im Knochenmark (D)** |
| 1 | >13 | >13 | 11.3 | 10.7 |
| 5'-Deoxy-5-fluoruridin | 5 | 6.4 | 5 - 10 | 5 - 10 |
| UFT | 1.5 | 2.5 | 1.1 | 1.5 - 2.9 |

* Die $ED_{50}$-Werte der Verbindung des Beispiels 1 von 5'-Deoxy-5-fluoruridin und von UFT am Tag 15 sind 0,15, 0,20 bzw. 0,086 mMol/kg/Tag.

** Die Toxizitäten wurden am Tag 10(A), 7(B), 14(C) und 14(D) ermittelt.

Antitumorwirksamkeit gegen Sarcoma 180, Meta A Fibrosarcoma und UV 2237 Fibrosarcoma

Die Antitumorwirksamkeit der Verbindung des vorliegenden Beispiels 1 wurde in drei Murintumormodellen mit derjenigen von 5'-Deoxy-5-fluoruridin und 5'-Deoxy-5-fluorcytidin verglichen. Mäuse wurden am Tag

0 subkutan mit Sarcoma 180, Meth A Fibrosarcoma und UV 2237 Fibrosarcoma inokuliert. Ab Tag 1 wurden die Versuchssubstanzen 7 mal den Mäusen oral verabreicht. Die Wirksamkeit wurde als therapeutischer Index ($ED_{max}/ED_{50}$) am Tag 14 nach Tumorinokulation gemessen. $ED_{max}$ ist die Dosis, bei der die Hemmung des Tumorwachstums maximal ist. Die Resultate sind in der Tabelle 5 angegeben.

Tabelle 5

| Wirksamkeit gegen Sarcom 180, Meth A Fibrosarcoma und UV 2237 Fibrosarcoma | | | |
|---|---|---|---|
| Verbindung (Beisp.-Nr.) | Therapeutischer Index | | |
| | S 180 | Meth A | UV 2237 |
| 1 | 2.3 | 2.0 | 4.8 |
| 5′-Deoxy-5-fluorcytidin | 2.0 | 1.2 | 1.0 |
| 5′-Deoxy-5-fluoruridin | 2.4 | 1.1 | 1.6 |

Akute Toxizität

Die akute Toxizität ($LD_{50}$) der Verbindungen der vorliegenden Beispiele 1, 5, 9, 24, 34, 46 und 47 beträgt mehr als 2000 mg/kg bei oraler Verabreichung an Mäusen.

Die folgenden Beispiele veranschaulichen die Erfindung.

Referenzbeispiel

a) 245 mg 5′-Deoxy-5-fluorcytidin, 354 mg t-Butyldimethylsilylchlorid und 284 mg Imidazol werden in 1,5 ml Dimethylformamid gelöst und 18 Stunden unter Stickstoff gerührt. Das Gemisch wird dann in Wasser geschüttet und mit Aethylacetat extrahiert. Der Extrakt wird mit Wasser gewaschen, über Natriumsulfat getrocknet und unter vermindertem Druck eingeengt. Man erhält 431 mg 2′,3′-Bis-O-(t-butyldimethylsilyl)-5′-deoxy-5-fluorcytidin, MS 473 ($M^+$).

b) Eine Lösung von 490 mg 5′-Deoxy-5-fluorcytidin, 418 mg p-Toluolsulfonsäure-monohydrat und 984 µl 2,2-Dimethoxypropan in 10 ml Aceton wird 1,5 Stunden gerührt. Der Lösung werden 900 mg Natriumbicarbonat zugesetzt und das Gemisch wird 4 Stunden gerührt. Der Niederschlag wird abfiltriert und mit Aceton gewaschen. Die Filtrate werden unter vermindertem Druck eingeengt. Der Rückstand wird über Silicagel mit Dichlormethan-Methanol gereinigt. Man erhält 570 mg 5′-Deoxy-5-fluor-2′,3′-O-isopropylidencytidin, MS 286 ($MH^+$), Smp. des Picrats 169-171 °C.

Beispiel 1

1(a) 9,46 g 2′,3′-Bis-O-(t-butyldimethylsilyl)-5′-deoxy-5-fluorcytidin, hergestellt nach Referenzbeispiel a), 3,48 g n-Buttersäureanhydrid und 2,93 g 4-Dimethylaminopyridin werden in 150 ml Methylenchlorid gelöst und über Nacht gerührt, dann mit Wasser gewaschen, über Natriumsulfat getrocknet und unter vermindertem Druck eingeengt. Man erhält 9,75 g $N^4$-Butyryl-2′,3′-bis-O-(t-butyldimethylsilyl)-5′-deoxy-5-fluorcytidin, MS 544 ($MH^+$).

1(b) 9,75 g des Produkts von Beispiel 1(a) werden in 80 ml Tetrahydrofuran enthaltend 80 mMol Tetrabutylammoniumfluorid gelöst und 1,5 Stunden gerührt. Nach Entfernung des Lösungsmittels unter vermindertem Druck wird der Rückstand über Silicagel mit Aethylacetat-Methanol gefolgt durch Umkristallisation aus Methanol gereinigt. Man erhält 4,5 g $N^4$-Butyryl-5'-deoxy-5-fluorcytidin, Smp. 156-157 °C; MS 316 ($MH^+$).

Die folgenden Verbindungen wurden in ähnlicher Weise wie in Beispiel 1 erhalten:

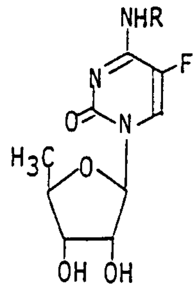

| Beispiel Nr. | R | Smp. °C | Umkristallisations- Lösungsmittel | MS |
|---|---|---|---|---|
| 2 | $-COCH_3$ | 157-159 | EtOH | 288 ($MH^+$) |
| 3 | 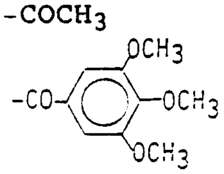 | 170-171 | $EtOAc-Et_2O$ | 440 ($MH^+$) |

Beispiel 4

4(a) Einer Lösung von 14,19 g 2',3'-Bis-O-(t-butyldimethylsilyl)-5'-deoxy-5-fluorcytidin in 150 ml trockenem Pyridin werden 3,84 g n-Butyrylchlorid unter Rühren zugetropft. Das Gemisch wird über Nacht gerührt. Pyridin wird unter vermindertem Druck entfernt und der Rückstand wird zwischen Wasser und Aethylacetat verteilt. Die Aethylacetatschicht wird mit Wasser gewaschen, über Natriumsulfat getrocknet und unter vermindertem Druck eingeengt. Der Rückstand wird über Silicagel mit n-Hexan-Aethylacetat gereinigt. Man erhält 15,32 g $N^4$-Butyryl-2',3'-bis-O-(t-butyldimethylsilyl)-5'-deoxy-5-fluorcytidin.

4(b) Das Produkt des Beispiels 4(a) wird in Analogie zu Beispiel 1(b) behandelt. Man erhält farblose Kristalle von $N^4$-Butyryl-5'-deoxy-5-fluorcytidin.

Die folgenden Verbindungen werden analog Beispiel 4 hergestellt:

| Beispiel Nr. | R | Smp. °C | Umkristallisations- Lösungsmittel | MS |
|---|---|---|---|---|
| 2 | $-CO(CH_2)_6CH_3$ | 106-107 | $Et_2O-MeOH$ | 372 ($MH^+$) |
| 6 | $-CO(CH_2)_7CH_3$ | (erhalten als amorphes Pulver) | | 386 ($MH^+$) |
| 7 | $-CO(CH_2)_{14}CH_3$ | 65-66 | MeOH | 484 ($MH^+$) |
| 8 | $-CO(CH_2)_{16}CH_3$ | 65-66 | MeOH | 512 ($MH^+$) |
| 9 | $-COCH(CH_3)_2$ | (erhalten als amorphes Pulver) | | 316 ($MH^+$) |

| Beispiel Nr. | R | Smp. °C | Umkristallisations-Lösungsmittel | MS |
|---|---|---|---|---|
| 10 | $-COC(CH_3)_3$ | (erhalten als amorphes Pulver) | | 330 ($MH^+$) |
| 11 | 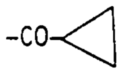 | 168-170 | EtOAc-MeOH | 314 ($MH^+$) |
| 12 | 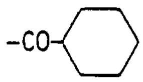 | (erhalten als amorphes Pulver) | | 356 ($MH^+$) |
| 13 | 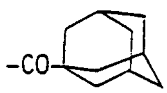 | (erhalten als amorphes Pulver) | | 408 ($MH^+$) |
| 14 | 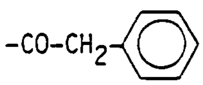 | (erhalten als amorphes Pulver) | | 364 ($MH^+$) |
| 15 | 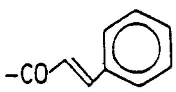 | 169-171 | EtOAc | 376 ($MH^+$) |
| 16 | 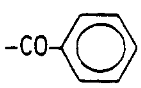 | 165-166 | EtOAc | 350 ($MH^+$) |
| 17 | 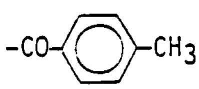 | 158-159 | MeOH | 363 ($M^+$) |
| 18 | 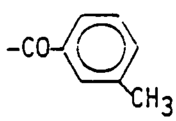 | 140-142 | EtOH | 364 ($MH^+$) |
| 19 | 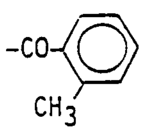 | 187-190 | EtOH | 364 ($MH^+$) |
| 20 | 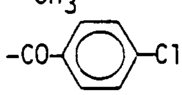 | 143-145 | EtOAc | 384 ($MH^+$) |
| 21 | 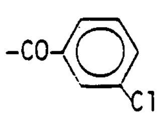 | 164-166 | EtOAc | 384 ($MH^+$) |

| Beispiel Nr. | R | Smp. °C | Umkristallisations-Lösungsmittel | MS |
|---|---|---|---|---|
| 22 | 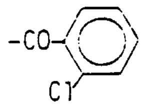 | 182-184 (Zers.) | MeOH | 384 ($MH^+$) |
| 23 | 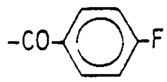 | 161-163 | MeOH | 368 ($MH^+$) |
| 24 | 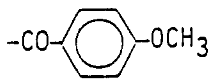 | 166-167 | EtOAc | 379 ($M^+$) |
| 25 | 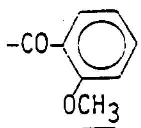 | 161-163 | $CH_2Cl_2$ | 380 ($MH^+$) |
| 26 | 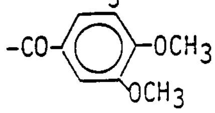 | 153-156 | MeOH | 410 ($MH^+$) |
| 27 | 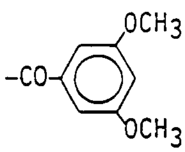 | 159-161 | $CH_2Cl_2$-MeOH | 410 ($MH^+$) |
| 28 | 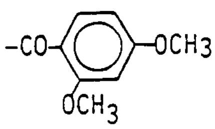 | 207-210 (Zers.) | EtOH | 410 ($MH^+$) |
| 29 | 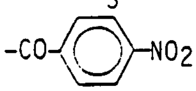 | 177-179 | $CH_2Cl_2$ | 395 ($MH^+$) |
| 30 | 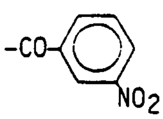 | 177-178 | EtOAc | 395 ($MH^+$) |
| 31 | 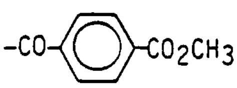 | 193-194 | MeOH | 408 ($MH^+$) |
| 32 | 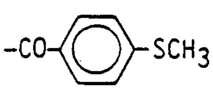 | 155-158 | MeOH | 396 ($MH^+$) |
| 33 | 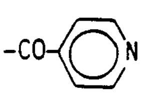 | 170-172 | EtOH | 351 ($MH^+$) |

| Beispiel Nr. | R | Smp. °C | Umkristallisations-Lösungsmittel | MS |
|---|---|---|---|---|
| 34 | 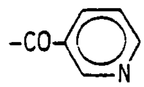 | 155-157 | MeOH | 351 ($MH^+$) |
| 35 | 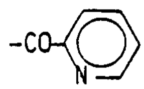 | 176-178 | EtOH | 351 ($MH^+$) |
| 36 | 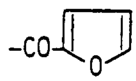 | 177-179 | EtOH | 340 ($MH^+$) |
| 37 | 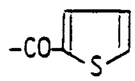 | 181-183 | EtOH | 356 ($MH^+$) |
| 38 | 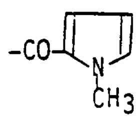 | 155-156 | EtOAc | 353 ($MH^+$) |
| 39 | 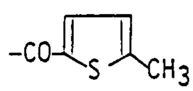 | 171-175 | MeOH | 370 ($MH^+$) |
| 40 | 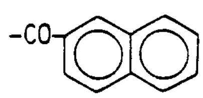 | 167-168 | $CH_2Cl_2$ | 400 ($MH^+$) |
| 41 | $-CO_2(CH_2)_7CH_3$ | 107-109 | $Et_2O$ | 402 ($MH^+$) |
| 42 | 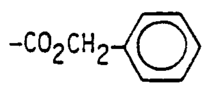 | 150-151 | MeOH | 380 ($MH^+$) |

Beispiel 43

735 mg 5'-Deoxy-5-fluorcytidin und 1,04 g Buttersäureanhydrid werden in 20 ml 75% wässrigem Dioxan gelöst und 18 Stunden gerührt. Nach Entfernung des Lösungsmittel wird der Rückstand durch Chromatographie über Silicagel gereinigt. Man erhält 420 mg $N^4$-Butyryl-5'-deoxy-5-fluorcytidin, Smp. 156-157 °C; MS 316 ($MH^+$).

Beispiel 44

(1) 4,9 g 5'-Deoxy-5-fluorcytidin und 5,58 ml Trimethylsilylchlorid werden in 50 ml trockenem Pyridin gelöst und 2 Stunden gerührt. Dem Reaktionsgemisch werden 2,09 ml Aethylchlorthioformat zugesetzt. Nach 2,5-stündigem Rühren wird das Pyridin unter vermindertem Druck abgedampft. Der Rückstand wird zwischen Wasser und Aethylacetat verteilt. Die organische Schicht wird mit Wasser gewaschen, über Natriumsulfat getrocknet und unter vermindertem Druck eingeengt. Dem Rückstand werden 5 g Zitronensäure und 80 ml Methanol zugesetzt. Das Gemisch wird 1 Stunde gerührt. Nach Entfernen des Lösungsmittels unter vermindertem Druck wird der Rückstand über Silicagel mit Methanol-Dichlormethan gefolgt durch Umkristallisation aus Dichlormethan gereinigt. Man erhält 2,66 g 5'-Deoxy-$N^4$-[(äthylthio)-carbonyl]-5-fluorcytidin, Smp. 138-139 °C (Zers.); MS 334 ($MH^+$).

(2)

(a) Einer Lösung von 1 g 5′-Deoxy-5-fluor-2′,3′-O-isopropylidencytidin erhalten nach Referenzbeispiel b) in 8 ml Pyridin werden 365 µl Aethylchlorthioformat bei 0 °C unter Rühren zugesetzt und das Gemisch wird über Nacht gerührt. Das Reaktionsgemisch wird unter vermindertem Druck eingeengt und der Rückstand wird zwischen Aethylacetat und Wasser verteilt. Die organische Schicht wird mit einer Natriumbicarbonatlösung und Wasser gewaschen und über Natriumsulfat getrocknet. Nach Entfernung des Lösungsmittels wird der Rückstand über Silicagel mit $CHCl_3$ gereinigt. Man erhält 510 mg 5′-Deoxy-$N^4$--[(äthylthio)carbonyl]-5-fluor -2',3'-O-isopropylidencytidin, MS 374 ($MH^+$).

(b) Einer Lösung von 150 mg des Produkts von Beispiel 44(2)(a) in 50% wässrigem Aethanol werden 150 mg Dowex 50 ($H^+$) zugesetzt und das Gemisch wird 4 Stunden unter Rühren bei 50-60 °C erhitzt. Das Dowex 50 wird abfiltriert und das Filtrat wird unter vermindertem Druck zur Trockene eingedampft. Der Rückstand wird über Silicagel mit $CHCl_3$-Aceton und dann durch Umkristallisation aus Dichlormethan gereinigt. Man erhält 5'-Deoxy-$N^4$-[(äthylthio)carbonyl]-5-fluorcytidin, Smp. 138-139 °C (Zers.); MS 334 ($MH^+$).

Die folgenden Verbindungen werden in Analogie zu Beispiel 44(1) erhalten:

| Beispiel Nr. | R | Smp. °C | Umkristallisations-Lösungsmittel | MS |
|---|---|---|---|---|
| 45 | $-COCH_2CH_3$ | 119-120 | $EtOAc-Et_2O$ | 302 ($MH^+$) |
| 46 | $-CO(CH_2)_3CH_3$ | 150-151 | EtOAc | 330 ($MH^+$) |
| 47 | $-COCH_2CH(CH_3)_2$ | 142-143 | EtOAc | 330 ($MH^+$) |

Beispiel 48

Eine Lösung von 0.42 g Piperonylsäure in trockenem Acetonitril (5 ml) enthaltend 0,36 ml Triäthylamin wird mit 0,37 ml Diäthylchlorphosphat I Stunde gerührt. Dem Reaktionsgemisch werden 1,0 g 2',3'-Bis-O-(t-butyldimethylsilyl)-5'--deoxy-5-fluorcytidin, hergestellt nach Beispiel a), 0,36 ml Triäthylamin und 0,05 g 4-Dimethylaminopyridin zugesetzt. Nach 12-stündigem Rühren wird das Acetonitril unter vermindertem Druck abgedampft. Der Rückstand wird zwischen Wasser und Aether verteilt. Die organische Schicht wird mit Wasser gewaschen, über Natriumsulfat getrocknet und unter vermindertem Druck eingeengt. Das erhaltene Pulver wird in 6,3 ml Tetrahydrofuran enthaltend 1,65 g Tetrabutylammoniumfluorid gelöst und das Reaktionsgemisch wird 1 Stunde gerührt. Nach Entfernung des Lösungsmittels unter vermindertem Druck wird der Rückstand über Silicagel mit Isopropanol-Dichlormethan und dann Umkristallisation aus Aethylacetat gereinigt. Man erhält 0,5 g 5′-Deoxy-5-fluor-$N^4$-piperonyloylcytidin, Smp. 124-125 °C, MS 394 ($MH^+$).

Die folgende Verbindung wird in Analogie zu Beispiel 48 erhalten:

| Beispiel Nr. | R | Smp. °C | Umkristallisations-Lösungsmittel | MS |
|---|---|---|---|---|
| 49 | $-COCH_2CH=CH_2$ | 137-138 | EtOAc | 314 ($MH^+$) |

Beispiel 50

0,355 g 3-Furancarbonsäure und 0,96 g 2,4,6-Triisopropylbenzolsulfonsäurechlorid werden in 5 ml trockenem Pyridin gelöst. Das Gemisch wird 1 Stunde gerührt. Dem Gemisch werden 1 g 2′,3′-Bis-O-(t-butyldimethylsilyl)-5′-deoxy-5-fluorcytidin, erhalten im Referenzbeispiel a), und 0,80 g 4-Dimethylaminopyridin zugesetzt. Nach 12 Stunden Rühren wird das Pyridin unter vermindertem Druck abgedampft. Nach Behandlung des Rückstands wie im Beispiel 48 erhält man 0,55 g 5′-Deoxy-5-fluor-$N^4$-(3-furoyl)cytidin, Smp. 173-174 °C (Aethanol); MS 340 ($MH^+$).

Die folgenden Verbindungen werden analog Beispiel 50 hergestellt:

| Beispiel Nr. | R | Smp. °C | Umkristallisations-Lösungsmittel | MS |
|---|---|---|---|---|
| 51 | 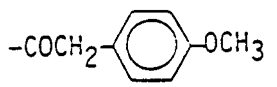 | (erhalten als amorphes Pulver) | | 394 ($MH^+$) |
| 52 | 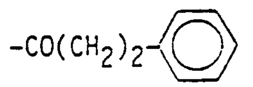 | 146-148 | EtOH | 378 ($MH^+$) |
| 53 | 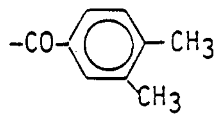 | 161-162 | EtOH | 378 ($MH^+$) |
| 54 | 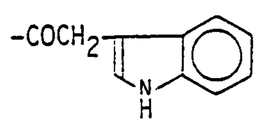 | (erhalten als amorphes Pulver) | | 403 ($MH^+$) |
| 55 | 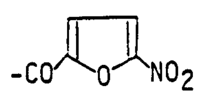 | 162-163 | EtOH | 385 ($MH^+$) |
| 55 | 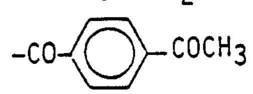 | 176-178 | EtOAc | 392 ($MH^+$) |

Beispiel 57

(a) Einer Lösung von 24,6 g 5′-Deoxy-5-fluoruridin in 150 ml trockenem Pyridin werden 24,5 ml Benzoylchlorid über 10 Minuten bei 0 °C Rühren zugetropft und das Gemisch wird 5 Stunden bei Raumtemperatur gerührt. Nach Entfernen des Pyridins unter vermindertem Druck wird der Rückstand zwischen Wasser und Aethylacetat verteilt. Die organische Schicht wird mit gesättigter Natriumbicarbonatlösung und Wasser gewaschen, über Natriumsulfat getrocknet und unter vermindertem Druck eingeengt. Der Rückstand wird aus Aethylacetat-n-Hexan umkristallisiert und man erhält 38,9 g 2′,3′-Di-O-benzoyl-5′-deoxy-5-fluoruridin, MS 455 ($MH^+$).
(b) Einem Gemisch von 0,8 ml N-Methylimidazol und 0,28 ml Phosphorylchlorid in 20 ml Acetonitril werden 500 mg 2′,3′-Di-O-benzoyl-5′-deoxy-5-fluoruridin bei 0 °C zugesetzt. Nach 1,5-stündigem Rühren werden 2,5 ml 28% Ammoniumhydroxid bei 0 °C zugesetzt und das Gemisch wird 1 Stunde bei Raumtemperatur gerührt. Das Acetonitril und das Ammoniak werden unter vermindertem Druck entfernt. Der Rückstand wird mit 1N HCl angesäuert und dann mit Aethylacetat extrahiert. Die organische Schicht wird mit Wasser gewaschen, über Natriumsulfat getrocknet und unter vermindertem Druck eingeengt. Der Rückstand wird aus Aethylacetat umkristallisiert. Man erhält 155 mg 2′,3′-Di-O-benzoyl-5′-deoxy-5-fluorcytidin, Smp. 192-194 °C; MS 476 $(M+Na)^+$.

Beispiel 58

(a) 0,57 ml eisgekühltem Essigsäureanhydrid werden 286 µl 99% Ameisensäure zugetropft. Die Lösung wird 15 Minuten bei 0 °C und 50 Minuten bei 50 °C gerührt und dann auf 0 °C abgekühlt. Der Lösung werden 473 mg 2′,3′-Bis-O-(t-butyldimethylsilyl)-5′-deoxy-5-fluorcytidin hergestellt nach Referenzbeispiel a) in 5 ml trockenem Pyridin bei 0 °C zugefügt. Das Reaktionsgemisch wird 10 Minuten bei 0 °C und 26 Stunden bei Raumtemperatur gerührt. Nach Entfernen des Lösungsmittels unter vermindertem Druck wird der Rückstand zwischen Wasser und Aethylacetat aufgeteilt. Die organische Schicht wird mit gesättigter Natriumbicarbonatlösung und Wasser gewaschen und über Natriumsulfat getrocknet. Das Aethylacetat wird unter vermindertem Druck abgedampft und der Rückstand wird über Silicagel mit n-Hexan-Aethylacetat und dann durch Umkristallisation aus n-Hexan-Aethylacetat gereinigt. Man erhält 144 mg 2′,3′-Bis-O-(t-butyldimethylsilyl)-5′-deoxy-5-fluor -$N^4$-formylcytidin¸ Smp. 188°C (Zers.); MS 502 ($MH^+$).

(b) Das Produkt des Beispiels 58(a) wird behandelt in Analogie zu Beispiel 1(b). Man erhält amorphes 5′-Deoxy-5-fluor-$N^4$-formylctidin¸ MS 274 ($MH^+$).

Beispiel 59

Einer Lösung von 245 mg 5′-Deoxy-5-fluorcytidin in 5 ml trockenem Pyridin werden unter Rühren bei 0°C 130 µl Benzoylchlorid zugesetzt. Das Gemisch wird 1 Stunde bei 0°C gerührt. Nach Entfernen des Lösungsmittels unter vermindertem Druck wird der Rückstand über Silicagel mit Dichlormethan-Methanol und dann durch Umkristallisation aus Aethylacetat gereinigt. Man erhält 51 mg 3′-O-Benzoyl-5′-deoxy-5-fluorcytidin, Smp. 127-129°C; MS 350 ($MH^+$).

Beispiel 60

Einer Lösung von 35 mg des Produkts von Beispiel 59 in 0,5 ml trockenem Pyridin werden 13,8 µl Trimethylsilylchlorid und nach 2-stündigem Rühren 12,6 µl Benzoylchlorid zugesetzt. Das Gemisch wird 1 Stunde gerührt. Nach Entfernen des Lösungsmittels unter vermindertem Druck wird der Rückstand in 0,5 ml Methanol gelöst. Der Lösung werden 15 mg Kaliumcarbonat zugesetzt und das Reaktionsgemisch wird 30 Minuten bei 0°C gerührt. Nach Entfernen des Lösungsmittels unter vermindertem Druck wird der Rückstand zwischen Wasser und Aethylacetat aufgeteilt. Die organische Schicht wird über Magnesiumsulfat getrocknet und unter vermindertem Druck eingeengt. Der Rückstand wird über Silicagel mit Dichlormethan-Methanol gereinigt. Man erhält 15 mg amorphes $N^4$,3′-O-Dibenzoyl-5′-deoxy-5-fluorcytidin, MS 454 ($MH^+$).

Beispiel 61

245 mg 5′-Deoxy-5-fluorcytidin, 400 µl Benzoylchlorid und 122 mg 4-Dimethylaminopyridin werden in 5 ml trockenem Pyridin gelöst. Nach 3-stündigem Rühren wird das Pyridin unter vermindertem Druck entfernt. Der Rückstand wird zwischen Aethylacetat und Wasser verteilt. Die Aethylacetatschicht wird über Magnesiumsulfat getrocknet und unter vermindertem Druck eingeengt. Der Rückstand wird aus Methanol umkristallisiert. Man erhält 280 mg $N^4$,2′-0,3′-O-Tribenzoyl-5′-deoxy-5-fluorcytidin, Smp. 158-160°C; MS 558 ($MH^+$).

Die nachfolgenden Beispiele veranschaulichen pharmazeutische Präparate auf der Basis einer Verbindung nach der vorliegenden Erfindung.

Beispiel A

Es werden in an sich bekannter Weise Gelatinesteckkapseln folgender Zusammensetzung hergestellt:

| | |
|---|---|
| $N^4$-Butyryl-5′-deoxy-5-fluorcytidin | 100 mg |
| Maisstärke | 20 mg |
| Titaniumdioxid | 385 mg |
| Magnesiumstearat | 5 mg |
| Film | 20 mg |
| PEG 6000 | 3 mg |
| Talk | 10 mg |
| Gesamtgewicht | 543 mg |

Beispiel B

Es werden in an sich bekannter Weise Tabletten folgender Zusammensetzung hergestellt:

| | |
|---|---|
| $N^4$-Butyryl-5′-deoxy-5-fluorcytidin | 100 mg |
| Lactose | 25 mg |
| Maisstärke | 20,2 mg |
| Hydroxypropylmethylcellulose | 4 mg |
| Magnesiumstearat | 0,8 mg |
| Film | 10 mg |
| PEG 6000 | 1,5 mg |
| Talk | 4,5 mg |
| Gesamtgewicht | 166 mg |

Beispiel C

(1) 5 g $N^4$-Butyryl-5′-deoxy-5-fluorcytidin werden in 75 ml destilliertem Wasser gelöst. Die Lösung wird einer bakteriologischen Filtration unterworfen und dann aseptisch in 10 sterilen Fläschchen geschüttet. Die Lösung wird dann trocken gefroren, sodass jedes Fläschchen 500 mg sterilen trockenen Feststoff enthält.

(2) Reines $N^4$-Butyryl-5′-deoxy-5-fluorcytidin in einer Menge von mg pro Fläschchen oder Ampulle wird versiegelt und heiss sterilisiert.

Vor der Verwendung werden den obigen trockenen Dosierungsformen ein steriles wässriges Lösungsmittel, wie Wasser für Injektion oder isotonische Natriumchloridlösung oder 5% Dextrose¸ für parenterale Verabreichung zugesetzt.

**Patentansprüche**

**1.** 5'-Deoxy-5-fluorcytidinderivate der allgemeinen Formel

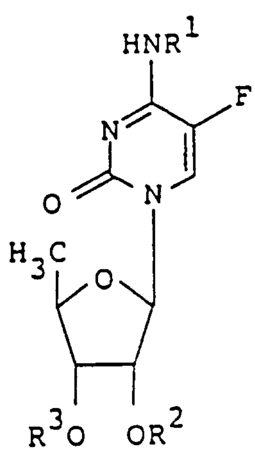

(I)

worin $R^1$, $R^2$ und $R^3$ unabhängig voneinander Wasserstoff oder eine unter physiologischen Bedingungen leicht abspaltbare Gruppe sind, mit der Bedingung, dass zumindest eines von $R^1$, $R^2$ und $R^3$ eine unter physiologischen Bedingungen leicht abspaltbare Gruppe ist, sowie Hydrate oder Solvate der Verbindungen der Formel I, wobei die leicht hydrolysierbaren Gruppen $R^1$, $R^2$ und $R^3$ in der Formel I eine der Formel

$R^4CO-$, $R^5OCO-$ oder $R^6SCO-$

worin $R^4$ Wasserstoff, Alkyl, Cycloalkyl, Oxoalkyl, Alkenyl, Aralkyl oder Aryl und $R^5$ und $R^6$ Alkyl oder Aralkyl sind.

2. Verbindungen nach Anspruch 1 aus der Gruppe der folgenden:
$N^4$-Acetyl-5'-deoxy-5-fluorcytidin,
5'-Deoxy-5-fluor-$N^4$-propionylcytidin,
$N^4$-Butyryl-5'-deoxy-5-fluorcytidin,
5'-Deoxy-5-fluor-$N^4$-isobutyrylcytidin,
5'-Deoxy-5-fluor-$N^4$-(2-methylbutyryl)cytidin,
5'-Deoxy-$N^4$-(2-äthylbutyryl)-5-fluorcytidin,
5'-Deoxy-$N^4$-(3,3-dimethylbutyryl)-5-fluorcytidin,
5'-Deoxy-5-fluor-$N^4$-pivaloylcytidin,
5'-Deoxy-5-fluor-$N^4$-valerylcytidin,
5'-Deoxy-5-fluor-$N^4$-isovalerylcytidin,
5'-Deoxy-5-fluor-$N^4$-(2-methylvaleryl)cytidin,
5'-Deoxy-5-fluor-$N^4$-(3-methylvaleryl)cytidin,
5'-Deoxy-5-fluor-$N^4$-(4-methylvaleryl)cytidin,
5'-Deoxy-5-fluor-$N^4$-hexanoylcytidin,
5'-Deoxy-5-fluor-$N^4$-heptanoylcytidin,
5'-Deoxy-5-fluor-$N^4$-octanoylcytidin,
5'-Deoxy-5-fluor-$N^4$-nonanoylcytidin,
5'-Deoxy-5-fluor-$N^4$-hexadecanoylcytidin,
$N^4$-Benzoyl-5'-deoxy-5-fluorcytidin,
5'-Deoxy-5-fluor-$N^4$-(4-methylbenzoyl)cytidin,
5'-Deoxy-5-fluor-$N^4$-(3-methylbenzoyl)cytidin,
5'-Deoxy-5-fluor-$N^4$-(2-methylbenzoyl)cytidin,
5'-Deoxy-$N^4$-(4-äthylbenzoyl)-5-fluorcytidin,
5'-Deoxy-$N^4$-(3,4-dimethylbenzoyl)-5-fluorcytidin,
5'-Deoxy-$N^4$-(3,5-dimethylbenzoyl)-5-fluorcytidin,
5'-Deoxy-5-fluor-$N^4$-(4-methoxybenzoyl)cytidin,
5'-Deoxy-$N^4$-(3,4-dimethoxybenzoyl)-5-fluorcytidin,
5'-Deoxy-$N^4$-(3,5-dimethoxybenzoyl)-5-fluorcytidin,
5'-Deoxy-5-fluor-$N^4$-(3,4,5-trimethoxybenzoyl)cytidin,
5'-Deoxy-5-fluor-$N^4$-(3,4,5-triäthoxybenzoyl)cytidin,
5'-Deoxy-$N^4$-(4-äthoxybenzoyl)-5-fluorcytidin,
5'-Deoxy-5-fluor-$N^4$-(4-propoxybenzoyl)cytidin,
5'-Deoxy-$N^4$-(3,5-diäthoxybenzoyl)-5-fluorcytidin,
$N^4$-(4-Chlorbenzoyl)-5'-deoxy-5-fluorcytidin,
5'-Deoxy-$N^4$-(3,4-dichlorbenzoyl)-5-fluorcytidin,
5'-Deoxy-$N^4$-(3,5-dichlorbenzoyl)-5-fluorcytidin,
5'-Deoxy-5-fluor-$N^4$-(4-nitrobenzoyl)cytidin,
5'-Deoxy-5-fluor-$N^4$-(4-methoxycarbonylbenzoyl)cytidin,
$N^4$-(4-Acetylbenzoyl)-5'-deoxy-5-fluorcytidin,
5'-Deoxy-5-fluor-$N^4$-(phenylacetyl)cytidin,
5'-Deoxy-5-fluor-$N^4$-(4-methoxyphenylacetyl)cytidin,
5'-Deoxy-5-fluor-$N^4$-nicotinoylcytidin,
5'-Deoxy-5-fluor-$N^4$-isonicotinoylcytidin,
5'-Deoxy-5-fluor-$N^4$-picolinoylcytidin,
5'-Deoxy-5-fluor-$N^4$-(2-furoyl)cytidin,
5'-Deoxy-5-fluor-$N^4$-(5-nitro-2-furoyl)cytidin,
5'-Deoxy-5-fluor-$N^4$-(2-thenoyl)cytidin,
5'-Deoxy-5-fluor-$N^4$-(5-methyl-2-thenoyl)cytidin,
5'-Deoxy-5-fluor-$N^4$-(1-methyl-2-pyrrolcarbonyl)cytidin,
5'-Deoxy-5-fluor-$N^4$-(3-indolylacetyl)cytidin,
$N^4$-(3-Butenoyl)-5'-deoxy-5-fluorcytidin,
3'-O-Benzoyl-5'-deoxy-5-fluorcytidin,
$N^4$,3'-O-Dibenzoyl-5'-deoxy-5-fluorcytidin und
5'-Deoxy-$N^4$-(äthylthio)carbonyl-5-fluorcytidin.

3. 5'-Deoxy-5-fluorcytidin-Derivate nach Anspruch 1 oder 2 aus der Gruppe der folgenden:
5'-Deoxy-5-fluor-$N^4$-octadecanoylcytidin,

$N^4$-Cyclopropancarbonyl-5'-deoxy-5-fluorcytidin,
$N^4$-Cyclohexancarbonyl-5'-deoxy-5-fluorcytldin,
$N^4$-(1-Adamantancarbonyl)-5'-deoxy-5-fluorcytidin,
5'-Deoxy-5-fluor-$N^4$-(2-methoxybenzoyl)cytidin,
5'-Deoxy-$N^4$-(2,4-dimethoxybenzoyl)-5-fluorcytidin,
5'-Deoxy-5-fluor-$N^4$-piperonyloylcytidin,
5'-Deoxy-5-fluor-$N^4$-(4-fluorbenzoyl)cytidin,
$N^4$-(2-Chlorbenzoyl)-5'-deoxy-5-fluorcytidin,
$N^4$-(3-Chlorbenzoyl)-5'-deoxy-5-fluorcytidin,
5'-Deoxy-5-fluor-$N^4$-(3-nitrobenzoyl)cytidin,
5'-Deoxy-5-fluor-$N^4$-[4-(methylthio)benzoyl]cytidin,
5'-Deoxy-5-fluor-$N^4$-(2-naphthoyl)cytidin,
5'-Deoxy-5-fluor-$N^4$-(3-furoyl)cytidin,
5'-Deoxy-5-fluor-$N^4$-(3-phenylpropionyl)cytidin,
$N^4$-Cinnamoyl-5'-deoxy-5-fluorcytidin,
2',3'-di-O-Benzoyl-5'-deoxy-5-fluorcytidin,
$N^4$,2'-0,3'-O-Tribenzoyl-5'-deoxy-5-fluorcytidin,
5'-Deoxy-5-fluor-$N^4$-(octyloxycarbonyl)cytidin,
$N^4$-(Benzyloxycarbonyl)-5'-deoxy-5-fluorcytidin und
5'-Deoxy-5-fluor-$N^4$-formylcytidin.

**4.** Die Verbindungen gemäss den Ansprüchen 1-3 zur Verwendung als Heilmittel, insbesondere als Antitumormittel.

**5.** Verfahren zur Herstellung der Verbindungen nach einem der Ansprüche 1-3, dadurch gekennzeichnet, dass man eine Verbindung der allgemeinen Formel

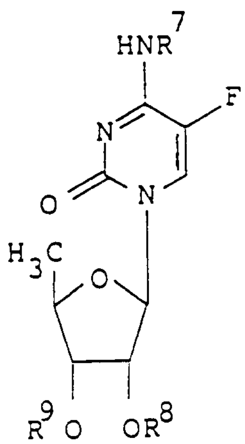

(II)

worin $R^7$ Wasserstoff oder eine Aminoschutzgruppe, $R^8$ und $R^9$ unabhängig voneinander Wasserstoff oder eine Hydroxyschutzgruppe oder $R^8$ und $R^9$ zusammen eine cyclische Hydroxyschutzgruppe sind,
mit einer Verbindung der allgemeinen Formel $XCOR^4$, worin X eine Abgangsgruppe und $R^4$ Wasserstoff, Alkyl, Cycloalkyl, Oxoalkyl, Alkenyl, Aralkyl oder Aryl ist, oder mit einer Verbindung der allgemeinen Formel $YCOR^{10}$, worin Y Halogen und $R^{10}$ eine Gruppe der Formel $R^5O$- oder $R^6S$-, in der $R^5$ und $R^6$ Alkyl oder Aralkyl sind, umsetzt und eine vorhandene Schutzgruppe abspaltet.

**6.** Pharmazeutische, insbesondere antitumor-wirksame Präparate auf der Basis einer Verbindung nach einem der Ansprüche 1-3.

**7.** Verwendung einer Verbindung nach einem der Ansprüche 1-3 zur Herstellung von Arzneimitteln zur Behandlung von Tumoren.

## Claims

1. 5'-deoxy-5-fluorocytidine derivatives of the general formula

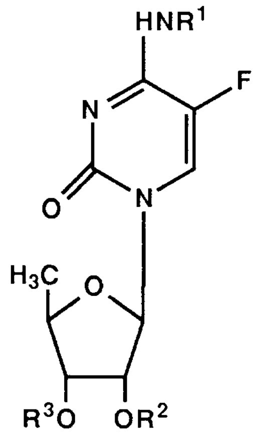

(I)

wherein $R^1$, $R^2$ and $R^3$ are each independently hydrogen or a group which is readily cleavable under physiological conditions, with the proviso that, at least one of $R^1$, $R^2$ or $R^3$ is a group which is readily cleavable under physiological conditions,
as well as hydrates or solvates of the compounds of formula I, whereby the readily hydrolyzable groups $R^1$, $R^2$ and $R^3$ in formula I are of the formula

$R^4CO$-, $R^5OCO$- or $R^6SCO$-

wherein $R^4$ is hydrogen, alkyl, cycloalkyl, oxoalkyl, alkenyl, aralkyl or aryl and $R^5$ and $R^6$ are alkyl or aralkyl.

2. Compounds according to claim 1 from the following group:
N4-Acetyl-5'-deoxy-5-fluorocytidine,
5'-deoxy-5-fluoro-N4-propionylcytidine,
N4-butyryl-5'-deoxy-5-fluorocytidine,
5'-deoxy-5-fluoro-N4-isobutyrylcytidine,
5'-deoxy-5-fluoro-N4-(2-methylbutyryl)cytidine,,
5'-deoxy-N4-(2-ethylbutyryl)-5-fluorocytidine,,
5'-deoxy-N4-(3,3-dimethylbutyryl)-5-fluorocytidine,
5'-deoxy-5-fluoro-N4-pivaloylcytidine,
5'-deoxy-5-fluoro-N4-valerylcytidine,
5'-deoxy-5-fluoro-N4-isovalerylcytidine,
5'-deoxy-5-fluoro-N4-(2-methylvaleryl)cytidine,
5'-deoxy-5-fluoro-N4-(3-methylvaleryl)cytidine,
5'-deoxy-5-fluoro-N4-(4-methylvaleryl)cytidine,
5'-deoxy-5-fluoro-N4-hexanoylcytidine,
5'-deoxy-5-fluoro-N4-heptanoylcytidine,
5'-deoxy-5-fluoro-N4-octanoylcytidine,
5'-deoxy-5-fluoro-N4-nonanoylcytidine,
5'-deoxy-5-fluoro-N4-hexadecanoylcytidine,,
N4-benzoyl-5'-deoxy-5-fluorocytidine,
5'-deoxy-5-fluoro-N4-(4-methylbenzoyl)cytidine,
5'-deoxy-5-fluoro-N4-(3-methylbenzoyl)cytidine,
5'-deoxy-5-fluoro-N4-(2-methylbenzoyl)cytidine,
5'-deoxy-N4-(4-ethylbenzoyl)-5-fluorocytidine,
5'-deoxy-N4-(3,4-dimethylbenzoyl)-5-fluorocytidine,
5'-deoxy-N4-(3,5-dimethylbenzoyl)-5-fluorocytidine,
5'-deoxy-5-fluoro-N4-(4-methylbenzoyl)cytidine,

5'-deoxy-N$^4$-(3,4-dimethoxybenzoyl)-5-fluorocytidine,
5'-deoxy-N$^4$-(3,5-dimethoxybenzoyl)-5-fluorocytidine,
5'-deoxy-5-fluoro-N$^4$-(3,4,5-trimethoxybenzoyl)cytidine,
5'-deoxy-5-fluoro-N$^4$-(3,4,5-triethoxybenzoyl)cytidine,
5'-deoxy-N$^4$-(4-ethoxybenzoyl)-5-fluorocytidine,
5'-deoxy-5-fluoro-N$^4$-(4-propoxybenzoyl)cytidine,
5'-deoxy-N$^4$-(3,5-diethoxybenzoyl)-5-fluorocytidine,
N$^4$-(4-chlorobenzoyl)-5'-deoxy-5-fluorocytidine,
5'-deoxy-N$^4$-(3,4-dichlorobenzoyl)-5-fluorocytidine,
5'-deoxy-N$^4$-(3,5-dichlorobenzoyl)-5-fluorocytidine,
5'-deoxy-5-fluoro-N$^4$-(4-nitrobenzoyl)cytidine,
5'-deoxy-5-fluoro-N$^4$-(4-methoxycarbonylbenzoyl)cytidine,
N$^4$-(4-acetylbenzoyl)-5'-deoxy-5-fluorocytidine,
5'-deoxy-5-fluoro-N$^4$ (phenylacetyl)cytidine,
5'-deoxy-5-fluoro-N$^4$-(4-methoxyphenylacetyl)cytidine,
5'-deoxy-5-fluoro-N$^4$-nicotinoylcytidine,
5'-deoxy-5-fluoro-N$^4$-isonicotinoylcytidine,
5'-deoxy-5-fluoro-N$^4$-picolinoylcytidine,
5'-deoxy-5-fluoro-N$^4$-(2-furoyl)cytidine,
5'-deoxy-5-fluoro-N$^4$-(5-nitro-2-furoyl)cytidine,
5'-deoxy-5-fluoro-N$^4$-(2-thenoyl)cytidine,
5'-deoxy-5-fluoro-N$^4$-(5-methyl-2-thenoyl)cytidine,
5'-deoxy-5-fluoro-N$^4$-(1-methyl-2-pyrrolecarbonyl)cytidine,
5'-deoxy-5-fluoro-N$^4$-(3-indolylacetyl)cytidine,
N$^4$-(3-butenoyl)-5'-deoxy-5-fluorocytidine,
3'-O-benzoyl-5'-deoxy-5-fluorocytidine,
N$^4$,3'-O-dibenzoyl-5'-deoxy-5-fluorocytidine and
5'-deoxy-N$^4$-(ethylthio)carbonyl-5-fluorocytidine.

**3.** Compounds according to claim 1 or 2 from the following group:
5'-Deoxy-5-fluoro-N$^4$-octadecanoylcytidine,
N$^4$-cyclopropanecarbonyl-5'-deoxy-5-fluorocytidine,
N$^4$-cyclohexanecarbonyl-5'-deoxy-5-fluorocytidine,
N$^4$-(1-adamantanecarbonyl-5'-deoxy-5-fluorocytidine,
5'-deoxy-5-fluoro-N$^4$-(2-methoxybenzoyl)cytidine,
5'-deoxy-N$^4$-(2,4-dimethoxybenzoyl)-5-fluorocytidine,
5'-deoxy-5-fluoro-N$^4$-piperonyloylcytidine,
5'-deoxy-5-fluoro-N$^4$-(4-fluorobenzoyl)cytidine,
N$^4$-(2-chlorobenzoyl)-5'-deoxy-5-fluorocytidine,
N$^4$-(3-chlorobenzoyl)-5'-deoxy-5-fluorocytidine,
5'-deoxy-5-fluoro-N$^4$-(3-nitrobenzoyl)cytidine,
5'-deoxy-5-fluoro-[N$^4$-(methylthio)benzoyl]cytidine,
5'-deoxy-5-fluoro-N$^4$-(2-naphthoyl)cytidine,
5'-deoxy-5-fluoro-N$^4$-(3-furoyl)cytidine,
5'-deoxy-5-fluoro-N$^4$-(3-phenylpropionyl)cytidine,
N$^4$-cinnamoyl-5'-deoxy-5-fluorocytidine,
2',3'-di-O-benzoyl-5'-deoxy-5-fluorocytidine,
N$^4$,2'-0,3'-O-tribenzoyl-5'-deoxy-5-fluorocytidine,
5'-deoxy-5-fluoro-N$^4$-(octyloxycarbonyl)cytidine,
N$^4$-(benzyloxycarbonyl)-5'-deoxy-5-fluorocytidine and
5'-deoxy-5-fluoro-N$^4$-formylcytidine.

**4.** The compounds according to claims 1-3 for use as medicaments, especially as antitumour agents.

**5.** A process for the manufacture of compounds according to any one of claims 1-3, characterized by reacting a compound of the general formula

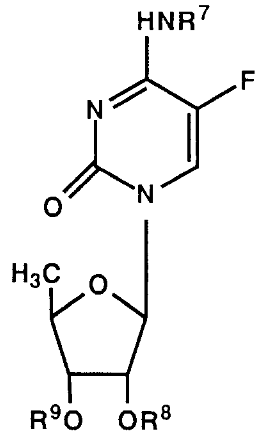

II

wherein $R^7$ is hydrogen or an amino protecting group, $R^8$ and $R^9$ are each independently hydrogen or a hydroxy protecting group or $R^8$ and $R^9$ together are a cyclic hydroxy protecting group, with a compound of the general formula $XCOR^4$, wherein X is a leaving group and $R^4$ is hydrogen, alkyl, cycloalkyl, oxoalkyl, alkenyl, aralkyl or aryl, or with a compound of the general formula $YCOR^{10}$, wherein Y is halogen and $R^{10}$ is a group of the formula $R^5O$- or $R^6S$-, in which $R^5$ and $R^6$ are alkyl or aralkyl, and cleaving off any protecting group present.

**6.** A pharmaceutical preparation, especially a preparation having antitumour activity, based on a compound according to any one of claims 1-3.

**7.** The use of a compound according to any one of claims 1-3 for the manufacture of medicaments for the treatment of tumours.

## Revendications

**1.** Dérivés de 5'-désoxy-5-fluorocytidine de formule générale

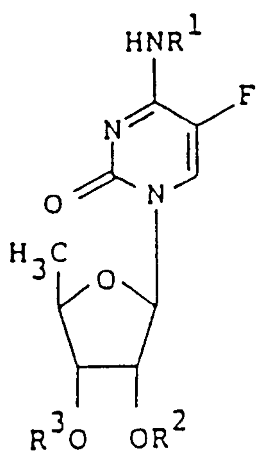

(I)

dans laquelle $R^1$, $R^2$ et $R^3$ sont indépendamment les uns des autres un atome d'hydrogène ou un groupe aisément séparable dans des conditions physiologiques, avec la condition qu'au moins un des radicaux $R^1$, $R^2$ et $R^3$ est un groupe aisément séparable dans des conditions physiologiques, et hydrates ou produits de solvatation des composés de formule I, les groupes aisément hydrolysables $R^1$, $R^2$ et $R^3$ dans la formule I étant un groupe de formule

$R^4CO$-, $R^5OCO$- ou $R^6SCO$-

formules dans lesquelles $R^4$ représente un atome d'hydrogène ou un groupe alkyle, cycloalkyle, oxoalkyle, alcényle, aralkyle ou aryle, et $R^5$ et $R^6$ représentent un groupe alkyle ou aralkyle.

**2.** Composés selon la revendication 1, choisis dans le groupe formé par les composés suivants:
$N^4$-acétyl-5'-désoxy-5-fluorocytidine,
5'-désoxy-5-fluoro-$N^4$-propionylcytidine,
$N^4$-butyryl-5'-désoxy-5-fluorocytidine,
5'-désoxy-5-fluoro-$N^4$-isobutyrylcytidine,
5'-désoxy-5-fluoro-$N^4$-(2-méthylbutyryl)cytidine,
5'-désoxy-$N^4$-(2-éthylbutyryl)-5-fluorocytidine,
5'-désoxy-$N^4$-(3,3-diméthylbutyryl)-5-fluorocytidine,
5'-désoxy-5-fluoro-$N^4$-pivaloylcytidine,
5'-désoxy-5-fluoro-$N^4$-valérylcytidine,
5'-désoxy-5-fluoro-$N^4$-isovalérylcytidine,
5'-désoxy-5-fluoro-$N^4$-(2-méthylvaléryl)cytidine,
5'-désoxy-5-fluoro-$N^4$-(3-méthylvaléryl)cytidine,
5'-désoxy-5-fluoro-$N^4$-(4-méthylvaléryl)cytidine,
5'-désoxy-5-fluoro-$N^4$-hexanoylcytidine,
5'-désoxy-5-fluoro-$N^4$-heptanoylcytidine,
5'-désoxy-5-fluoro-$N^4$-octanoylcytidine,
5'-désoxy-5-fluoro-$N^4$-nonanoylcytidine,
5'-désoxy-5-fluoro-$N^4$-hexadécanoylcytidine,
$N^4$-benzoyl-5'-désoxy-5-fluorocytidine,
5'-désoxy-5-fluoro-$N^4$-(4-méthylbenzoyl)cytidine,
5'-désoxy-5-fluoro-$N^4$-(3-méthylbenzoyl)cytidine,
5'-désoxy-5-fluoro-$N^4$-(2-méthylbenzoyl)cytidine,
5'-désoxy-$N^4$-(4-éthylbenzoyl)-5-fluorocytidine,
5'-désoxy-$N^4$-(3,4-diméthylbenzoyl)-5-fluorocytidine,
5'-désoxy-$N^4$-(3,5-diméthylbenzoyl)-5-fluorocytidine,
5'-désoxy-5-fluoro-$N^4$-(4-méthoxybenzoyl)cytidine,
5'-désoxy-$N^4$-(3,4-diméthoxybenzoyl)-5-fluorocytidine,
5'-désoxy-$N^4$-(3,5-diméthoxybenzoyl)-5-fluorocytidine,
5'-désoxy-5-fluoro-$N^4$-(3,4,5-triméthoxybenzoyl)cytidine,
5'-désoxy-5-fluoro-$N^4$-(3,4,5-triéthoxybenzoyl)cytidine,
5'-désoxy-$N^4$-(4-éthoxybenzoyl)-5-fluorocytidine,
5'-désoxy-5-fluoro-$N^4$-(4-propoxybenzoyl)cytidine,
5'-désoxy-$N^4$-(3,5-diéthoxybenzoyl)-5-fluorocytidine,
$N^4$-(4-chlorobenzoyl)-5'-désoxy-5-fluorocytidine,
5'-désoxy-$N^4$-(3,4-dichlorobenzoyl)-5-fluorocytidine,
5'-désoxy-$N^4$-(3,5-dichlorobenzoyl)-5-fluorocytidine,
5'-désoxy-5-fluoro-$N^4$-(4-nitrobenzoyl)cytidine,
5'-désoxy-5-fluoro-$N^4$-(4-méthoxycarbonylbenzoyl)cytidine,
$N^4$-(4-acétylbenzoyl)-5'-désoxy-5-fluorocytidine,
5'-désoxy-5-fluoro-$N^4$-(phénylacétyl)cytidine,
5'-désoxy-5-fluoro-$N^4$-(4-méthoxyphénylacétyl)cytidine,
5'-désoxy-5-fluoro-$N^4$-nicotinoylcytidine,
5'-désoxy-5-fluoro-$N^4$-isonicotinoylcytidine,
5'-désoxy-5-fluoro-$N^4$-picolinoylcytidine,
5'-désoxy-5-fluoro-$N^4$-(2-furoyl)cytidine,
5'-désoxy-5-fluoro-$N^4$-(5-nitro-2-furoyl)cytidine,
5'-désoxy-5-fluoro-$N^4$-(2-thénoyl)cytidine,
5'-désoxy-5-fluoro-$N^4$-(5-méthyl-2-thénoyl)cytidine,
5'-désoxy-5-fluoro-$N^4$-(1-méthyl-2-pyrrolcarbonyl)cytidine,
5'-désoxy-5-fluoro-$N^4$-(3-indolylacétyl)cytidine,
$N^4$-(3-buténoyl)-5'-désoxy-5-fluorocytidine,
3'-O-benzoyl-5'-désoxy-5-fluorocytidine,
$N^4$,3'-O-dibenzoyl-5'-désoxy-5-fluorocytidine et
5'-désoxy-$N^4$-(éthylthio)carbonyl-5-fluorocytidine.

3. Dérivés de 5'-désoxy-5-fluorocytidine selon la revendication 1 ou 2, choisis dans le groupe formé par les composés suivants:
5'-désoxy-5-fluoro-$N^4$-octadécanoylcytidine,
$N^4$-cyclopropanecarbonyl-5'-désoxy-5-fluorocytidine,
$N^4$-cyclohexanecarbonyl-5'-désoxy-5-fluorocytidine,
$N^4$-(1-adamantanecarbonyl)-5'-désoxy-5-fluorocytidine,
5'-désoxy-5-fluoro-$N^4$-(2-méthoxybenzoyl)cytidine,
5'-désoxy-$N^4$-(2,4-diméthoxybenzoyl)-5-fluorocytidine,
5'-désoxy-5-fluoro-$N^4$-pipéronyloylcytidine,
5'-désoxy-5-fluoro-$N^4$-(4-fluorobenzoyl)cytidine,
$N^4$-(2-chlorobenzoyl)-5'-désoxy-5-fluorocytidine,
$N^4$-(3-chlorobenzoyl)-5'-désoxy-5-fluorocytidine,
5'-désoxy-5-fluoro-$N^4$-(3-nitrobenzoyl)cytidine,
5'-désoxy-5-fluoro-$N^4$-[4-(méthylthio)benzoyl]cytidine,
5'-désoxy-5-fluoro-$N^4$-(2-naphtoyl)cytidine,
5'-désoxy-5-fluoro-$N^4$-(3-furoyl)cytidine,
5'-désoxy-5-fluoro-$N^4$-(3-phénylpropionyl)cytidine,
$N^4$-cinnamoyl-5'-désoxy-5-fluorocytidine,
2',3'-di-O-benzoyl-5'-désoxy-5-fluorocytidine,
$N^4$,2'-0,3'-O-tribenzoyl-5'-désoxy-5-fluorocytidine,
5'-désoxy-5-fluoro-$N^4$-(octyloxycarbonyl)cytidine,
$N^4$-(benzyloxycarbonyl)-5'-désoxy-5-fluorocytidine et
5'-désoxy-5-fluoro-$N^4$-formylcytidine.

4. Composés selon les revendications 1 à 3, pour utilisation en tant que médicaments, en particulier en tant que médicaments antitumoraux.

5. Procédé pour la préparation des composés selon l'une des revendications 1 à 3, caractérisé en ce que l'on fait réagir un composé de formule générale

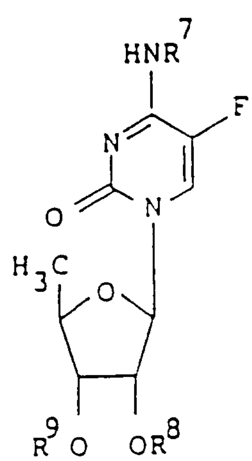

(II)

dans laquelle $R^7$ est un atome d'hydrogène ou un groupe protecteur de groupe amino, $R^8$ et $R^9$ sont indépendamment l'un de l'autre un atome d'hydrogène ou un groupe protecteur de groupe hydroxy, ou $R^8$ et $R^9$ forment ensemble un groupe cyclique protecteur de groupe hydroxy,
avec un composé de formule générale $XCOR^4$, dans laquelle X représente un groupe partant et $R^4$ représente un atome d'hydrogène ou un groupe alkyle, cycloalkyle, oxoalkyle, alcényle, aralkyle ou aryle, ou avec un composé de formule générale $YCOR^{10}$ , dans laquelle Y est un atome d'halogène et $R^{10}$ est un groupe de formule $R^5O$- ou $R^6S$-, dans lequel $R^5$ et $R^6$ représentent un groupe alkyle ou aralkyle, et on élimine un groupe protecteur présent.

6. Compositions pharmaceutiques, en particulier à action antitumorale, à base d'un composé selon l'une des revendications 1 à 3.

**7.** Utilisation d'un composé selon l'une des revendications 1 à 3, pour la fabrication de médicaments destinés au traitement de tumeurs.